# EUROPEAN PATENT SPECIFICATION

(11) **EP 3 342 360 B1**
(45) Date of publication and mention of the grant of the patent: **29.04.2020**
(21) Application number: 16838231.5
(22) Date of filing: 26.02.2016
(51) Int. Cl.: A61B 17/64

(54) **EXTERNAL FIXING DEVICE USED FOR TREATMENT OF CALCANEAL FRACTURE**
EXTERNE BEFESTIGUNGSVORRICHTUNG ZUR BEHANDLUNG VON FERSENBEINBRÜCHEN
DISPOSITIF DE FIXATION EXTERNE UTILISÉ POUR LE TRAITEMENT DE FRACTURE DU CALCANÉUS

(30) Priority: 26.08.2015 CN 201510531826; 26.08.2015 CN 201520651348 U
(43) Date of publication of application: 04.07.2018
(73) Proprietor: J&L Medical Technology Co., Ltd., Shunyi District Beijing 101399 (CN)
(72) Inventor: LIANG, Xiangdang, Beijing 100853 (CN)
(74) Representative: Neusser, Sebastian
(86) International application number: PCT/CN2016/074667
(87) International publication number: WO 2017/031968

(56) References cited:
- WO-A2-2008/011018
- CN-A- 103 829 993
- CN-A- 104 116 551
- CN-A- 105 213 002
- CN-U- 204 890 145
- CN-Y- 2 569 754
- US-A1- 2013 018 424
- US-A1- 2014 066 931
- US-B1- 6 572 620

## Description

### TECHNICAL FIELD

The present disclosure relates to the technical field of medical appliances and more particularly to an external fixing device for treating a fracture of a calcaneal.

### BACKGROUND

In the related art, a major approach for treating a calcaneal fracture is surgical treatment. The purpose of treatment is to fix a fracture site by internal fixing method or external fixing method. Specifically, the internal fixing method using steel plates and screws is a major clinical treatment approach at present. However, it has such disadvantages as large surgical trauma and consequently slow healing of an incision, which may easily cause complications such as infections. Moreover, the surgical procedure is complicated and one or more additional surgeries may be further needed so as to achieve a healing effect. Using an external fixing frame is also one approach for treating a calcaneal fracture. Due to heavy weight, complex operation, bad fixing effect and ease of loosening of the external fixing frame, application of external fixing frame is greatly restricted. WO 2008/011018 A2 discloses an external fixing device according to the preamble of claim 1. This document discloses a fully contoured calcaneal plate comprising a superior plate segment, an anterior plate segment and an inferior plate segment, all adjacent and adjoining and defining an interior portion having at least one fenestration, a plurality of screw holes which accept screws for attachment of the plate to a bone, an anterior tail portion extending inferiorly to superiorly.

### SUMMARY

The present disclosure is intended to overcome at least one of technical problems existing in the prior art. For this purpose, an example of the present disclosure provides an external fixing device for treating a fracture of a calcaneal as mentioned in the independent claim. The external fixing device is of relatively light weight and convenient to operate and it can be used for the left and right foot.

An external fixing device for treating a fracture of a calcaneal of the present disclosure includes an external fixing plate and a plurality of screws. Where, a shape of the external fixing plate is adapted to match a contour of the calcaneus, a hollow portion that extends through the external fixing plate along thickness direction is formed in the external fixing plate and a plurality of threaded holes are formed on the external fixing plate; one end of each of the screws matches a corresponding one of the threaded holes by thread fit and the other end of each of the screws is suitable to screw into the calcaneus.

For the external fixing device for treating the fracture of the calcaneal provided in an example of the present disclosure, by providing the hollow portion on the external fixing device, the weight of the external fixing device may be reduce so as to facilitate observing and treating a wound. Further, the external fixing device is easy to operate and can achieve a good fixing effect with little damage.

According to an example of the present disclosure, the threaded holes may be spaced from each other along a circumference of the hollow portion.

According to an example of the present disclosure, the threaded holes may be uniformly distributed along the circumference of the hollow portion.

According to an example of the present disclosure, a shape of the hollow portion may be adapted to match a contour of the calcaneus.

According to an example of the present disclosure, an inner circumferential wall of each of the threaded holes is provided with two types of internal threads having opposite thread directions.

According to an example of the present disclosure, a diameter of each of the screws may range from 3.0 mm to 3.5 mm.

According to an example of the present disclosure, the diameter of each of the screws may be 3.0 mm.

According to an example of the present disclosure, a number of the screws may range from 12 to 16.

According to an example of the present disclosure, a positioning hole corresponding to one of the threaded holes may be formed on the calcaneus by drilling with a Kirschner wire. According to an example of the present disclosure, the external fixing plate may be a flat plate.

According to an example of the present disclosure, the external fixing plate may be a stainless steel plate, a titanium alloy plate or an aluminium alloy plate.

Additional aspects and advantages of the present disclosure will partially be given in the following description, and partially become apparent from the following description or be known by practicing the present disclosure.

### BRIEF DESCRIPTION OF THE DRAWINGS

The above and/or additional aspects and advantages of the present disclosure will become apparent and understandable in the descriptions that are made to the examples in combination with following drawings.
FIG. 1 is a schematic diagram illustrating an external fixing plate of an external fixing device for treating a fracture of a calcaneal according to an example of the present disclosure.
FIG.2 is a schematic diagram illustrating a screw of an external fixing device for treating a fracture of a calcaneal according to an example of the present disclosure.

### DETAILED DESCRIPTION OF THE EMBODIMENTS

Examples of the present disclosure will be described in detail below. Instance of these examples are illustrated in the accompanying drawings. The same or similar numerals always represent the same or similar components or components having the same or similar functions. The examples described below by referring to the accompanying drawings are illustrative and only used for explaining the present disclosure and shall not be understood as limiting the present disclosure.

In the descriptions of the present disclosure, it is to be understood that orientations and positional relationships indicated by terms such as center, upper, lower, front, back, left, right, vertical, horizontal, top, bottom, internal and external are orientations or positional relationships illustrated in the accompanying drawings and are only used for convenience and simplicity of descriptions rather than for indicating or implying devices or components therein must have a particular orientation or be constructed or operated in a particular orientation and therefore shall not be understood as limiting the present disclosure. It is to be noted that the meaning of "a plurality of' refers to two or more unless otherwise stated in the descriptions of the present disclosure.

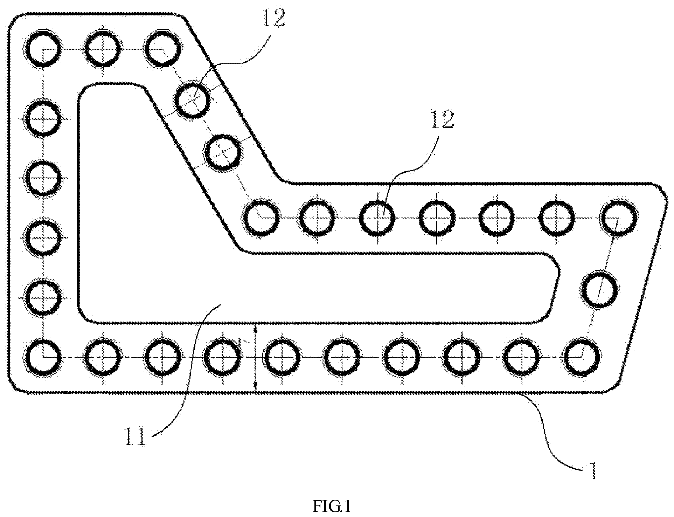

An external fixing device for treating a fracture of a calcaneal in an example of the present disclosure will be described below by referring to FIG. 1.

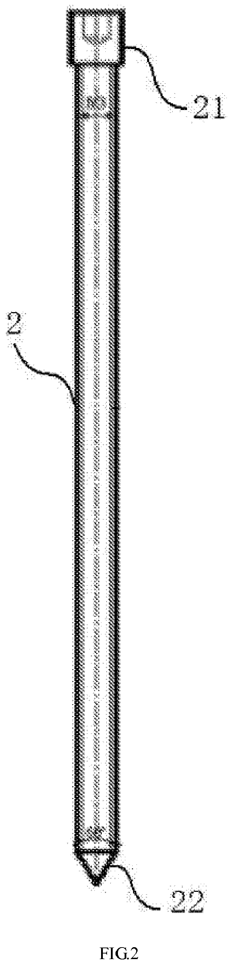

As shown in FIGs. 1 and 2, the external fixing device for treating a fracture of a calcaneal in an example of the present disclosure may include an external fixing plate 1 and a plurality of screws 2.

A shape of the external fixing plate 1 may be adapted to match a contour of the calcaneus. For example, the shape of the external fixing plate may be designed based on the contour of the calcaneus. Thus, a fracture site of the calcaneus and the calcaneus itself may be fixed as well as possible. For example, in the example shown in FIG 1, the external fixing plate 1 is substantially formed into an L shape.

Alternatively, the external fixing plate 1 may be a flat plate. Thus, processing technology of producing the external fixing plate 1 is simple and producing cost is reduced.

A hollow portion 11 that extends through the external fixing plate 1 along thickness direction may be formed in the external fixing plate 1. Since swelling usually accompanies bone fracture, the hollow portion 11 may be provided such that a wound may be observed through the hollow portion 11. Thus, specific situations of the wound may be monitored in real time. Also, the wound may be sterilized or treated through dressing change conveniently. Further, weight of external fixing plate 1 may be reduced by providing the hollow portion 11 in the external fixing plate 1 so that user experience may be improved. In addition, the external fixing plate 1 is designed to be a closed loop based on the contour of the calcaneus, which effectively guarantees strength of the external fixing plate 1.

A plurality of threaded holes 12 may be formed on the external fixing plate 1. As shown in FIG 1, each of the threaded holes 12 may extend through the external fixing plate 1 along the thickness direction of the external fixing plate 1 and an internal thread may be formed on an inner wall of each of the threaded holes 12.

One end 21 of the screw 2, for example, an upper end shown in FIG. 2, may match a corresponding threaded hole 12 by thread fit and the other end 22 of the screw 2, for example, a lower end shown in FIG 2, may be suitable to screw into the calcaneus. For example, the two ends of the screw 2 may both be provided with an external thread. The external thread of the end 21 of the screw 2 may fixedly engage the external fixing plate 1 and the external thread of the end 22 of the screw 2 is suitable to fix the bone. A portion between the two ends of the screw 2 may be formed with a smooth external surface so that the portion is suitable for contacting skin or a soft tissue.

The external fixing plate 1 may be a stainless steel plate, a titanium alloy plate, an aluminum alloy plate or the like. That is, the external fixing plate 1 may be made with light materials such as stainless steel, titanium alloy or aluminum alloy and have a small volume so that the weight of the external fixing plate 1 may be further reduced, thereby relieving pain of a patient and facilitating recovery of the patient.

In actual practice, the end 21 of the screw 2 may match the threaded hole 12 by thread fit such that the screw 2 is connected with the external fixing plate 1. The end 22 of the screw 2 may be screwed into a calcaneus, for example, a calcaneus of a human body, to fix a fracture site. During the process, the screw 2 may be screwed through the threaded hole 12.

Since the external fixing device for treating a fracture of a calcaneal in an example of the present disclosure is used outside a human body and falls within external fixing scope, resulted trauma is rendered small with less bleeding (for example, an incision reduced by folds to extending only 4 centimeters along a limb), thereby the complications of the incision may be greatly reduced. Further, the external fixing device is fixed in an external fixing manner, therefore, a subsequent surgical operation is not required, thereby avoiding risks caused by the subsequent surgical operation and saving medical costs for a patient. In addition, with the screws 2, an entire structure of the external fixing device is more secure and volume of the external fixing device is smaller.

The weight of the external fixing device for treating a fracture of a calcaneal in an example of the present disclosure is reduced by providing the hollow portion 11, thereby facilitating the recovery of a patient. Further, the external fixing device is convenient to operate and has good fixing effect.

An external fixing device for treating a fracture of a calcaneal according to an example of the present disclosure is described below in combination with FIGs.1 and 2.

As shown in FIGs. 1 and 2, the external fixing device for treating a fracture of a calcaneal may include an external fixing plate 1 and screws 2. The external fixing plate 1 may be designed into a shape as shown in FIG. 1 based on a contour of a calcaneus. A hollow portion 11 extending through the external fixing plate 1 may be formed in the external fixing plate 1 and a shape of the hollow portion 11 is adapted to match the contour of the calcaneus. On one hand, observation of a foot and operations such as sterilizing and changing dressings for a wound may be facilitated; on the other hand, strength of the entire external fixing plate 1 is substantially uniformly distributed so that the entire structural strength of the external fixing plate 1 is guaranteed.

A plurality of threaded holes 12 may be spaced from each other along a circumference of the hollow portion 11. Preferably a plurality of threaded holes 12 are uniformly distributed along the circumference of the hollow portion 11. Thus, reliability of fixing strength may be guaranteed. Diameter of each of the screws may range from 3.0 mm to 3.5 mm. Specific value for the diameter may be set based on actual demand so as to satisfy actual requirements better. It is appreciated that a greater number of threaded holes 12 may be arranged on the external fixing plate 1, that is, density of the threaded holes 12 may be larger, which may allow a doctor to select fixing positions for the screws 2 freely based on actual needs. Further, the weight of the external fixing plate 1 may be further reduced. According to an example of the present disclosure, the external fixing plate 1 may be fixed to a calcaneus using a plurality of thin screws (i.e. screws 2 that have smaller cross-section area). For example, a diameter of each of the screws 2 may be 3.0 mm and a number of the screws 2 may range from 12 to 16. Thus, trauma is smaller and fixing may be achieved with higher reliability.

Alternatively, a positioning hole corresponding to one of the threaded holes 12 may be formed on the calcaneus by drilling with a Kirschner wire.

A surgical procedure of the external fixing device for treating a fracture of a calcaneal in an example of the present disclosure is as follows: first, reduce a fracture of a calcaneal in a manner of open or closed percutanecous reduction by leverage; then fix the external fixing plate 1 at the fracture of the calcaneus temporarily using Kirschner wires; next, confirm whether the position of the external fixing plate 1 is correct under radioscopy, for example, X-ray radioscopy. If the position is correct, drill on the calcaneus with the Kirschner wires to form one or more abovementioned positioning holes, then take out the Kirschner wires and screw the screws 2 for fixing. If the position is not correct, take out the Kirschner wires used for temporary fixing, adjust the position of the external fixing plate 1 and re-fix the external fixing plate 1 at the fracture of the calcaneus temporarily using Kirschner wires.

Each of the threaded holes may be screwed with a sleeve. Drilling with Kirschner wire through the sleeve may cause damage and complications of screw paths are as less as possible, thereby improving safety of surgical operation.

For example, for a fracture with obvious joint surface collapse, open reduction of joint surface and bone grafting may be performed firstly and the joint surface may be then fixed with brad nails, absorbable nails and the like. Subsequently, the incision may be closed and the external fixing plate 1 may be placed. At this time, it may be important to screw in some screws 2 below the joint surface so as to increase support for the joint surface.

For another example, for an avulsion fracture at a tubercle of a calcaneus, it may be important to screw in some screws 2 in the tubercle region of the calcaneus so as to fix the fracture and prevent the calcaneus from being dislocated at the fracture under pulling force of a calcaneal tendon. If necessary, all the threaded holes 12 in a region adjacent to the tubercle of the calcaneus may be screwed with the screws 2 to increase a holding force for the fracture portion.

Two types of internal threads having opposite thread directions may be provided on an inner circumferential wall of each of the threaded holes 12. Thus, the screws 2 may be screwed in in two directions so that the external fixing plate 1 may be used for a left foot as well as a right foot, thereby increasing versatility of the external fixing plate 1.

The external fixing device for treating a fracture of a calcaneal in an example of the present disclosure is characterized by micro-invasion and no need for subsequent surgical operation, etc., which effectively reduces incision complications in a calcaneal fracture treatment.

In the descriptions of the present disclosure, descriptions for terms such as one example, some examples, illustrative examples, example, specific example and so on are intended to mean that specific characteristics, structures, materials or features described in combination with the examples are included in at least one example of the present disclosure. In the descriptions of the present disclosure, the illustrative descriptions for the above terms are not necessarily intended to refer to the same examples. Further, the described specific characteristics, structures, materials or features may be combined in a proper way in any one or more examples.

Although the examples of the present disclosure are already illustrated and described, the persons skilled in the art may understand that various changes, modifications, substitutions and variations may be made to these examples without departing from the principle and the spirit of the present disclosure. The scope of protection of the present disclosure is limited by the appended claims.

## Claims

1. An external fixing device for treating a fracture of a calcaneal, wherein the external fixing device (1) comprises:
an external fixing plate (1) provided with a hollow portion (11) and a plurality of threaded holes (12), wherein a shape of the external fixing plate (1) is adapted to match a contour of a calcaneus, and the hollow portion (11) extends through the external fixing plate (1) along a thickness direction of the external fixing plate (1); and
a plurality of screws (2), wherein one end (21) of each of the screws (2) matches one of the threaded holes (12) by thread fit and the other end (22) of each of the screws (2) is suitable to screw into the calcaneus, **characterized in that** two types of internal threads that differ in thread direction are formed on an inner circumferential wall of each of the threaded holes (12).

2. The external fixing device for treating the fracture of the calcaneal according to claim 1, **characterized in that**,
the threaded holes (12) are spaced from each other along a circumference of the hollow portion (11).

3. The external fixing device for treating the fracture of the calcaneal according to claim 2, **characterized in that**,
the threaded holes (12) are uniformly distributed along the circumference of the hollow portion (11).

4. The external fixing device for treating the fracture of the calcaneal according to any one of claims 1 to 3, **characterized in that**,
a shape of the hollow portion (11) is adapted to match the contour of the calcaneus.

5. The external fixing device for treating the fracture of the calcaneal according to any one of claims 1 to 4, **characterized in that**,
a diameter of each of the screws (2) ranges from 3.0 mm to 3.5 mm.

6. The external fixing device for treating the fracture of the calcaneal according to claim 5, **characterized in that**,
the diameter of each of the screws (2) is 3.0 mm.

7. The external fixing device for treating the fracture of the calcaneal according to any one of claims 1 to 6, **characterized in that**,
a number of the screws (2) ranges from 12 to 16.

8. The external fixing device for treating the fracture of the calcaneal according to any one of claims 1 to 7, **characterized in that**,
the external fixing plate (1) is a flat plate.

9. The external fixing device for treating the fracture of the calcaneal according to any one of claims 1 to 8, **characterized in that**,
the external fixing plate (1) is a stainless steel plate, a titanium alloy plate or an aluminum alloy plate.

## Patentansprüche

1. Externe Befestigungsvorrichtung zum Behandeln einer Fraktur eines Calcaneus, wobei die externe Befestigungsvorrichtung (1) umfasst:
eine externe Befestigungsplatte (1), welche mit einem hohlen Abschnitt (11) und mehreren Gewindelöchern (12) versehen ist, wobei eine Form der externen Befestigungsplatte (1) ausgestaltet ist, um mit einer Kontur eines Calcaneus übereinzustimmen, und wobei sich der hohle Abschnitt (11) durch die externe Befestigungsplatte (1) entlang einer Dickenrichtung der externen Befestigungsplatte (1) erstreckt; und
mehrere Schrauben (2), wobei ein Ende (21) jeder der Schrauben (2) mit ihrem Gewinde zu einem der Gewindelöcher (12) passt und das andere Ende (22) jeder der Schrauben (2) geeignet ist, um in den Calcaneus geschraubt zu werden, **dadurch gekennzeichnet, dass** zwei Typen von Innengewinden, welche sich in einer Gewinderichtung unterscheiden, auf einer inneren Umfangswand von jedem der Gewindelöcher (12) ausgebildet sind.

2. Externe Befestigungsvorrichtung zum Behandeln einer Fraktur des Calcaneus nach Anspruch 1,
**dadurch gekennzeichnet,**
**dass** die Gewindelöcher (12) voneinander entlang eines Umfangs des hohlen Abschnitts (11) beabstandet sind.

3. Externe Befestigungsvorrichtung zum Behandeln einer Fraktur des Calcaneus nach Anspruch 2,
**dadurch gekennzeichnet,**
**dass** die Gewindelöcher (12) gleichmäßig entlang des Umfangs des hohlen Abschnitts (11) verteilt sind.

4. Externe Befestigungsvorrichtung zum Behandeln einer Fraktur des Calcaneus nach einem der Ansprüche 1 bis 3,
**dadurch gekennzeichnet,**
**dass** eine Form des hohlen Abschnitts (11) ausgestaltet ist, um mit der Kontur des Calcaneus übereinzustimmen.

5. Externe Befestigungsvorrichtung zum Behandeln einer Fraktur des Calcaneus nach einem der Ansprüche 1 bis 4,
**dadurch gekennzeichnet,**
**dass** ein Durchmesser von jeder der Schrauben (2) in einem Bereich von 3,0 mm bis 3,5 mm liegt.

6. Externe Befestigungsvorrichtung zum Behandeln einer Fraktur des Calcaneus nach Anspruch 5,
**dadurch gekennzeichnet,**
**dass** der Durchmesser von jeder der Schrauben (2) 3,0 mm ist.

7. Externe Befestigungsvorrichtung zum Behandeln einer Fraktur des Calcaneus nach einem der Ansprüche 1 bis 6,
**dadurch gekennzeichnet,**
**dass** eine Anzahl der Schrauben (2) in einem Bereich von 12 bis 16 liegt.

8. Externe Befestigungsvorrichtung zum Behandeln einer Fraktur des Calcaneus nach einem der Ansprüche 1 bis 7,
**dadurch gekennzeichnet,**
**dass** die externe Befestigungsplatte (1) eine flache Platte ist.

9. Externe Befestigungsvorrichtung zum Behandeln einer Fraktur des Calcaneus nach einem der Ansprüche 1 bis 8,
**dadurch gekennzeichnet,**
**dass** die externe Befestigungsplatte (1) eine Edelstahlplatte, eine Titanlegierungsplatte oder eine Aluminiumlegierungsplatte ist.

## Revendications

1. Dispositif de fixation externe destiné au traitement d'une fracture d'un calcanéus, dans lequel le dispositif de fixation externe (1) comprend :
une plaque de fixation externe (1) pourvue d'une partie creuse (11) et d'une pluralité de trous filetés (12), dans lequel une forme de la plaque de fixation externe (1) est à même de s'adapter à un contour d'un calcanéus, et la partie creuse (11) s'étend à travers la plaque de fixation externe (1) dans le sens de l'épaisseur de la plaque de fixation externe (1) ; et
une pluralité de vis (2), dans lequel une première extrémité (21) de chacune des vis (2) s'adapte à l'un des trous filetés (12) par ajustement de filets et l'autre extrémité (22) de chacune des vis (2) convient pour se visser dans le calcanéus, **caractérisé en ce que** deux types de filets internes qui diffèrent dans le sens de filetage sont formés sur une paroi circonférentielle interne de chacun des trous filetés (12).

2. Dispositif de fixation externe destiné au traitement de la fracture du calcanéus selon la revendication 1, **caractérisé en ce que** :
les trous filetés (12) sont espacés les uns des autres le long d'une circonférence de la partie creuse (11).

3. Dispositif de fixation externe destiné au traitement de la fracture du calcanéus selon la revendication 2, **caractérisé en ce que** :
les trous taraudés (12) sont uniformément distribués le long de la circonférence de la partie creuse (11).

4. Dispositif de fixation externe destiné au traitement de la fracture du calcanéus selon l'une quelconque des revendications 1 à 3, **caractérisé en ce que** :
une forme de la partie creuse (11) est à même de s'adapter au contour du calcanéus.

5. Dispositif de fixation externe destiné au traitement de la fracture du calcanéus selon l'une quelconque des revendications 1 à 4, **caractérisé en ce que** :
un diamètre de chacune des vis (2) se situe dans une plage de 3,0 mm à 3,5 mm.

6. Dispositif de fixation externe destiné au traitement de la fracture du calcanéus selon la revendication 5, **caractérisé en ce que** :
le diamètre de chacune des vis (2) est de 3,0 mm.

7. Dispositif de fixation externe destiné au traitement de la fracture du calcanéus selon l'une quelconque des revendications 1 à 6, **caractérisé en ce que** :
un nombre des vis (2) se situe dans une plage de 12 à 16.

8. Dispositif de fixation externe destiné au traitement de la fracture du calcanéus selon l'une quelconque des revendications 1 à 7, **caractérisé en ce que** :
la plaque de fixation externe (1) est une plaque plate.

9. Dispositif de fixation externe destiné au traitement de la fracture du calcanéus selon l'une quelconque des revendications 1 à 8, **caractérisé en ce que** :
la plaque de fixation externe (1) est une plaque d'acier inoxydable, une plaque d'alliage de titane ou une plaque d'alliage d'aluminium.
